# EUROPEAN PATENT APPLICATION

(11) **EP 2 592 090 A1**
(43) Date of publication of application: **15.05.2013**
(21) Application number: 11188776.6
(22) Date of filing: 11.11.2011
(51) Int. Cl.: C07K 9/00, C07K 1/18, A61K 38/14

(54) **Process of purification of teicoplanin**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The invention provides an inexpensive process for isolation of teicoplanin from a fermentation culture of *Actinoplanes teichomyceticus* in a grade for pharmaceutical application with substantial removal of colours. Applying optimal combination of cation and anion exchange techniques together with further improved purification affords a unique decoloured teicoplanin material.
According to some embodiments, the glycopeptide antibiotic is eluted from the ion exchange column at a pH from 5 to below 9.

## Description

### Field of the Invention

The present invention relates to the field of chemical technology, more specifically to a novel and effective way of purification of the antibiotic teicoplanin.

### Background of the invention

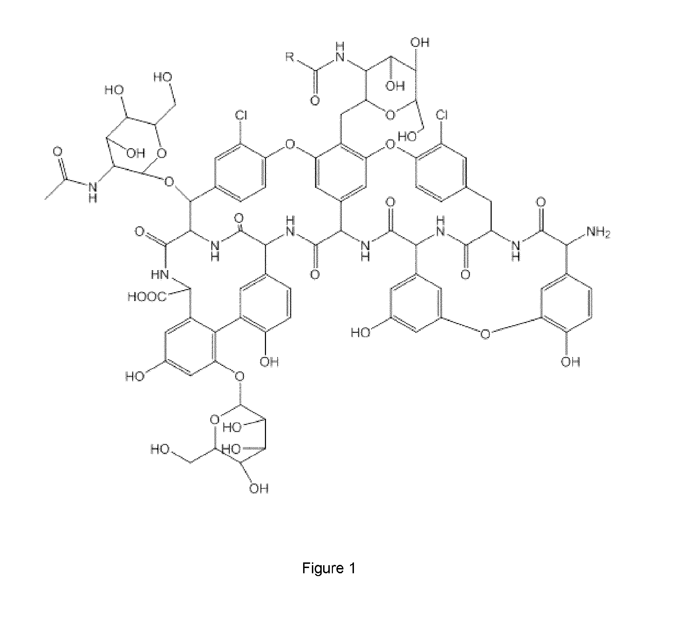

Teicoplanin is a commonly used term for a group of glycopeptide antibiotics from the vancomycin-ristocetin family, produced by a fungal microorganism such as e.g. *Actinoplanes teichomyceticus.* Teicoplanin is firstly disclosed in J. Antibiotics 31 (1978), p. 170-177 as a mixture of three factors named teichomycin A₁, A₂ and A₃. Factor A₂, later called teicoplanin A₂, was identified as the major component, which was isolated by column chromatography on Sephadex LH-20™. Teicoplanin A₂ was later recognized as a mixture of five molecules (J. Antibiotics 37 (1984), p. 615-620), which were soon elucidated as structures differing in the nature of an acyl-aliphatic side chain (J. Am. Chem. Soc. 106 (1984), 4895-4902). An exemplified and typical compound representation of teicoplanin is illustrated in Fig. 1 (R may denote various C₁₀-C₁₁-alkyl residues). These five A₂ compounds constitute teicoplanin together with another major component (A₃) and some minor components (e.g. Rₛ-1 to Rₛ-4). The mixture of these components in a limited ratio variation is marketed as Targocid® for treating infections by gram-positive bacteria. Besides vancomycin and vancomycin-like antibiotics it still remains a key active compound against highly resistent microorganisms.

A number of efforts have been made to separate teicoplanin from a culture broth of the microorganism and to purify teicoplanin to reach pharmaceutical grade. According to the first publication in US 4239751, a culture broth is divided into a mycelial cake and a filtrate. After treatment of the cake both fractions were extracted with butanol at acidic pH and the crude fermentation product was precipitated after partial removal of the solvent. The precipitates were collected, purified on Sephadex LH20 column and on an acidic ion exchange resin, such as Amberlite IR-120 and Dowex 50, with final isolation of product by precipitation at 4°C. However, this process is a laboratory trial of isolation with several disadvantages for industrial use in view of complexity, quality, costs and yields.

In order to improve yields of fermentation in such self-inhibited production of teicoplanin the product was tried to be continuously removed by adding water-miscible organic solvents, such as acetone, n-propanol, and acetonitrile (Korean Pat. No. 367890), or by adding a strongly acidic cation exchange resin, such as Dow XFS-43278.00 and Diaion SK-1 02 (Korean Pat. No. 118034) to a culture broth. But such processes still need large volumes of organic solvents to extract teicoplanin causing handling problems and environmental pollution.

US patent application 4,845,194 reports a process for purification of teicoplanin involving a cation exchange technique without prior pH adjustment. Teicoplanin is eluted from the cation exchange resin using a solution having a pH value of from about 9 to about 12.

Further progress in the isolation of teicoplanin was made by discovery of the efficient binding of teicoplanin to hydrophobic resins, firstly disclosed in EP 0241758, using a special polyamide resin and further upgrading the purification by an extraction process of teicoplanin from mycelium at alkaline pH as a simplification of primary disclosed processes (EP 0479086). However, the purity of teicoplanin was not higher than 85 % and the decolourisation was insufficient.

The introduction of commercial hydrophobic resins, preferably applied as a column rather than in suspension treatment, represented a further progress. The publication J. Biochem. 275 (2000), p. 6201-6206 describes the use of a commercial hydrophobic resin Dianion HP2 MGL™ with further elution using NaOH solution. However, the approach required the continuous neutralization of the eluting solution in order to prevent epimerisation of the alkali-sensitive product. Even if accompanied with other special approaches in order to obtain pure teicoplanin, in view of a mere 50 to 60 % (w/w) of purity this laboratory process is rather suitable for purposes other than use as a pharmaceutical ingredient.

Korean Pat. No. 321304 discloses a neutral adsorption resin and a lectin-immobilized affinity chromatography step using resins selected from the group consisting of Amberlite XAD 16, Diaion HP 20, silica gel, and activated carbon. However, in case that the filtered culture broth extracted from a basic solution is adsorbed to a resin, such as Diaion HP 20, a substantial amount of teicoplanin is lost in the adsorption step, and the purity of teicoplanin eluted by a methanol concentration gradient is very low. Moreover, it is necessary to remove methanol in order to apply the solution to a lectin-immobilized resin. Furthermore, it is not desirable to apply the strategy in large scale production because of the cost of lectin-immobilized resins. According to Korean pat. appl. 2003/017067 teicoplanin of a culture broth is adsorbed to a porous adsorption resin, the porous adsorption resin is washed with diluted hydrochloric acid and teicoplanin is desorbed from the adsorption resin using a mixed solution of water and acetone. The eluting solution containing teicoplanin is concentrated by vacuum distillation, treated with an activated carbon, and subjected to a precipitation process, and thereby teicoplanin is purified. Again, the process is insufficient for a scale up due to the irreversible adsorption of teicoplanin to the resin reducing the yield and due to the use of large amounts of solvent . Furthermore, it is difficult to purify teicoplanin to 95 area % or higher employing only a single process that uses porous adsorption resins.

Alternatively, some other studies have included reverse phase resins to separate and purify teicoplanin from the culture broth. For example, a process, published in Chromatographia 24 (1987) p. 295-301 proposed purifying teicoplanin using a Lichrosorb™ resin, but the use of large amounts of acetonitrile and the frequent exchange of resin may considerably enhance production costs. Two other alternatives accomplished the chromatographic methods on reverse phase resins by additional manners of purification. For example, Korean pat. appl. 2003/092504 describes extraction of fermentation products with aqueous acetone followed by chromatography on silane coated silica gel, adsorption on activated carbon in the step of isolating and ultra-filtration. US 7405267 recites a process of purifying teicoplanin from a culture broth, which includes a primary purifying step using a synthetic adsorbent, a secondary purifying using a cation-exchange resin, a catalytic resin, or a chelate resin, a tertiary purifying step using a reverse phase resin, and a final lyophilisation step. Accordingly, the use of more expensive reverse phase alone cannot avoid the use of additional adsorbents, so it does not justify the additional costs. In summary, all above described methods offer only partial solutions, which do not solve the complexity of isolation and purification of teicoplanin. The patent EP 1735337B1 has offered the most comprehensive solution for isolation and purification of teicoplanin until now. It went back to the adsorption method on hydrophobic resins describing the following key steps of purification and isolation of teicoplanin:
- separation of mycelium from dissolved teicoplanin in a filtrate,
- adsorption of roughly purified teicoplanin to porous adsorption resins from neutral 10-40 % aqueous C₁-C₄-alcohols or C₃-C₄-ketones medium of pH 6.5 to 7.5, followed by elution with 40 to 90 % aqueous C₁-C₄-alcohols with careful salt concentration control,
- adsorption of impurities to activated carbon using diluted aqueous alcohol solution, roughly calculated from aqueous methanol with max. 30 %
- desalination by adsorption to porous resins followed by elution with 40 to 90 % aqueous C₁-C₄-alcohols
- ultrafiltration on membranes with pores size from 3 - 100 kDa at 0 - 4 kPa of pressure using diluted aqueous alcoholic solutions of purified teicoplanin in concentration less than 20 %
- precipitation of teicoplanin.

Although this process may represent the best literature option of isolation of teicoplanin, its reproduction surprisingly shows considerable drawbacks:
- desorption from porous resins are not complete (maximally 87.6 %);
- diminishing of size pores in ultrafiltration surprisingly reduces passage of teicoplanin through membranes although the pores are considerably larger than teicoplanin molecules radius, while using larger pores reduces selectivity, so several cycles of ultrafiltration is needed enhancing solvent medium amount to huge volumes;
- removal of coloured impurities remains the main problem, because colours are not completely removed neither in the step adsorption on resins nor during decolourisation on charcoal and ultrafiltration.

Hence, there is still a need for an efficient comprehensive procedure of isolation of teicoplanin of pharmaceutical grade.

### Summary of the invention

The present invention provides the following aspects, subject-matters and preferred embodiments, which respectively taken alone or in combination, further contribute to solving the object of the present invention:
1. A process for preparing purified teicoplanin, the process comprising subjecting teicoplanin to combined purification steps involving both, a cation exchange technique and an anion exchange technique.
2. A process according to item 1 comprising the steps, in the following indicated order, of either
   a) providing teicoplanin in solution;
   b) binding teicoplanin from the solution to a cation exchange resin, followed by eluting with an eluant;
   c) binding teicoplanin to an anion exchange resin, followed by eluting with an eluant, and
   d) obtaining purified teicoplanin;
   or
   a') providing crude teicoplanin in solution;
   b') binding teicoplanin from the solution to an anion exchange resin, followed by eluting with an eluant;
   c') binding teicoplanin to a cation exchange resin, followed by eluting with an eluant, and d') obtaining purified teicoplanin.
3. A process for preparing purified teicoplanin, the process comprising subjecting teicoplanin to a cation or to an anion exchange technique, wherein either a cation or an anion exchange resin having teicoplanin bound thereto is treated with an eluant having a pH value in the range from 5 to below 9.
4. The process according to item 3, wherein the eluant has a pH value in the range from 5.5 to 8.5, preferably from 6 to 8.5.
5. The process according to any one of the preceding items, wherein teicoplanin is previously provided by a process, which includes at least one of the following steps, preferably a combination of the indicated steps, of
   a1) incubating a fermentation culture of a strain of *Actinoplanes teichomyceticus* producing teicoplanin compounds, followed by removal of undissolved material by filtration, ultrafiltration or centrifugation;
   a2) adsorbing teicoplanin to a hydrophobic adsorption resin, optionally followed by washing the resin, and elution from the resin by a mixture of water and water-miscible solvent, wherein preferably the water-miscible solvent is methanol;
   a3) treating a teicoplanin sample with charcoal;
   a4) concentrating and desalinating a solution of teicoplanin by ultrafiltration on a membrane with a cut-off of at least 1000 Da.
6. The process according to item 5, wherein teicoplanin is isolated after the respective step of any one of the items a1) to a4) by evaporation and/or precipitation with an antisolvent, followed by redissolving the obtained crude teicoplanin in solid form in a solvent to thereby provide teicoplanin in solution.
7. The process according to any one of the preceding items, wherein coloured teicoplanin of any source, including a commercial source, is used as a starting material.
8. The process according to any one of the preceding items, wherein a cation ion exchange resin is used, which is a macroreticular weak acid cation exchange resin selected from the group consisting of Amberlite^{®} IRC, Amberlite^{®} FPC, Diaion^{®} WK and Purolite^{®} C trademark groups.
9. The process according to item 8, wherein the cation ion exchange resin is selected from the group consisting of Amberlite IRC50™ , Amberlite FPC 3500™ and Diaion WK 100™.
10. The process according to item 8 or 9, wherein the cation ion exchange resin is Amberlite IRC50™ in a protonic form.
11. The process according to any one of the preceding items, wherein an anion exchange resin is used, which is selected from the group consisting of Amberlite^{®} FPA, Dowex^{®} Marathon, Dowex^{®} Upcore, Diaion^{®} WA and Purolite^{®} A trademark groups.
12. The process according to item 11, wherein the anion exchange resin is selected from the group consisting of Amberlite FPA51™, Amberlite FPA55™, Dowex Marathon WBA™, Purolite A830™.
13. The process according to item 11 or 12, wherein the anion exchange resin is Purolite A830™.
14. The process according to any one of the preceding items, wherein, after performing either cation exchange or anion exchange, the teicoplanin-containing sample is treated by at least one further method of purification before proceeding with the respectively other ion exchange technique.
15. The process according to item 14, wherein the at least one further method of purification comprises at least one selected from the group consisting of a treatment of the solution with charcoal; removing biomacromolecules by ultrafiltration using a membrane with a cut-off of at least 5000 Da; concentrating and desalinating the solution by ultrafiltration on a membrane with a cut-off of at least 1000 Da; removing salts and biomacromolecules by an absorption/desorption process on a hydrophobic adsorption resins; extraction of impurities by a water-immiscible organic solvent; concentrating the solution, optionally with partial exchange of solvents.
16. The process according to any one of items 5 to 15, wherein the hydrophobic adsorption resin is a polystyrene or polyacrylate crosslinked non-ionic macroreticular resin having a pore size of 20 to 500 A.
17. The process according to any one of items 5 to 16, wherein the hydrophobic adsorption resin has a pore size of 100 to 500 A.
18. The process according to any one of items 5 to 17, wherein the hydrophobic adsorption resin is selected from the group consisting of Hypersol-Macronet^{®}, Dowex^{®} Optipore, Amberlite^{®} XAD16, Amberlite^{®} XAD 1600, Amberlite^{®} XAD 1180, Diaion^{®} HP20, Diaion^{®} Sepabeads SP207, Dianion^{®} HP2MG.
19. The process according to any one of items 5 to 18, wherein the hydrophobic adsorption resin is selected from the group consisting of Amberlite^{®} XAD16, Amberlite^{®} XAD 1600, Amberlite^{®} XAD 1180.
20. The process according to any one of the preceding items, wherein purified teicoplanin is isolated by any method selected from spray drying; liophilisation; evaporation of the solvent, preferably at reduced pressure; or precipitation using an antisolvent and/or with pH adjustment.
21. The process according to item 20, wherein the antisolvent is a water-miscible solvent selected from the group consisting of C₃-C₄-alcohols, C₃-C₄-ketones or C₂-C₄ nitriles.
22. The process according to item 20 or 21, wherein the antisolvent is acetone.
23. The process according to any one of items 20 to 22, wherein the volume of the antisolvent is 5-15 times larger than the volume of the teicoplanin solution.
24. The process according to any one of items 20 to 23, wherein the volume of the antisolvent is 10 times larger than the volume of the teicoplanin solution.
25. The process according to any one of the preceding items, wherein the pH of the solution is optionally adjusted in any step of the process or in a plurality of steps of the process to 6.5 to 7.5 by NaOH or HCl.
26. The process according to any one of the preceding items, wherein the teicoplanin obtained as a final product is characterized by a significantly reduced content of coloured material in comparison to crude teicoplanin.
27. The process according to any one of the preceding items, wherein in the cation exchange technique an eluant comprises 10-40 % of a water-miscible solvent in water.
28. The process according to any one of the preceding items, wherein in the anion exchange technique an eluant comprises 10-50 % of a water-miscible solvent in water.
29. The process according to item 27 or 28, wherein the eluant is a methanolic solution.
30. The process according to any one of the preceding items, wherein, in the cation and/or the anion exchange technique, an eluant comprises an organic buffer.
31. The process according to item 30, wherein the organic buffer is selected from the group consisting of Tris/HCl, Bicine, Tricine or HEPES.
32. The process according to items 1 to 29, wherein, in the cation and/or the anion exchange technique, an eluant comprises a solution of a salt of a weak acid.
33. The process according to any one of the preceding items, wherein, in the cation and/or the anion exchange technique, an eluant comprises at least one ionic compound.
34. The process according to item 33, wherein the ionic compound is a cation selected from alkali metal cations; earth alkali metal cations; ammonium; mono-, di-, tri- or tetra-substituted ammonium, in which substituents are identical or different and selected from C₁-C₁₆-alkyl, aryl, substituted C₁-C₄-alkyl.
35. The process according to item 33 or 34, wherein the ionic compound comprised in the eluant is an alkali metal cation.
36. The process according to item 33, wherein the ionic compound is an anion of a weak acid selected from the group consisting of phosphate; hydrogen phosphate; dihydrogen phosphate; carbonate; hydrogen carbonate; borate; organic C₁-C₁₆-containing linear or branched alkyl carboxylate, dicarboxylate, tricarboxylate or tetracarboxylate, wherein alkyl is optionally substituted with 1-3 hydroxy groups, 1-2 amino groups, a mercapto or methylmercapto group or a phenyl group.
37. The process according to item 36, wherein the anion is acetate.
38. The process according to item 32 or 33, wherein, in the cationic and/or the anion exchange technique, the eluant comprises a solution of an alkali salt of a lower aliphatic acid.
39. The process according to item 32 or 33, wherein, in the cation and/or the anion exchange technique, the eluant comprises a solution of sodium acetate in a concentration range of 0.01-0.5 mol/L.
40. The process according to item 32 or 33, wherein, in the cation and/or the anion exchange technique, the eluant comprises a 0.1M solution of sodium acetate.
41. Teicoplanin characterized by having a transmittance of more than 80 %, at wavelength 405 nm upon dissolution in water at a concentration of 40 g/l.
42. The teicoplanin according to item 41, wherein the teicoplanin is obtained by a process according to any of items 1 to 40.
43. A pharmaceutical composition comprising the teicoplanin of item 41 or 42.
44. A process for manufacturing a pharmaceutical composition comprising providing teicoplanin as defined in item 41 or 42, and mixing the provided teicoplanin with at least one pharmaceutically acceptable excipient or carrier.
45. A process for manufacturing a pharmaceutical composition, the process comprising the process according to any one of items 1 to 40 and mixing isolated purified teicoplanin with at least one pharmaceutically acceptable excipient or carrier.

### Brief description of the drawings

- Figure 1:: Illustrates an exemplified and typical compound representation of teicoplanin;
- Figure 2:: Schematically shows an oxidative deamination reaction of teicoplanin
- Figure 3:: Schematically shows an overview of a whole process for production of teicoplanin.

### Detailed description of the invention

The present invention is now described in more detail by preferred embodiments and examples, which are however presented for illustrative purpose only and shall not be understood as limiting the scope of the present invention in any way.

The present invention provides an inexpensive process for isolation of teicoplanin from a fermentation culture of a suitable fungal microorganism producing teicoplanin such as e.g. *Actinoplanes teichomyceticus* in a grade for pharmaceutical application with substantial removal of colours. Applying optimal combination of cation and anion exchange techniques together with further improved purification affords a unique decoloured teicoplanin material.

The term "teicoplanin" as used in the context of the invention relates to a glycopeptide antibiotic that consists of a mixture of compounds or to the single components of said mixture. The single components of the mixture typically comprise five major components (A₂-1, A₂-2, A₂-3, A₂-4, A₂-5), a core glycopeptide A₃-1 and minor components such as RS-1, RS-2, RS-3 and RS-4. Explicit reference is made to the description of the background of the invention recited herein. The content ratio of the single components may not be constant and can vary according to the used strain, culturing conditions or the purification process applied.

The fermentation culture of e.g. *Actinoplanes teichomyceticus* used for the production of teicoplanin typically is an intensely brown to almost black coloured broth, which can be gradually decolourised through a selected technological procedure by adsorption and chromatographic methods known to a skilled person, in order to obtain a final product that is still considerably coloured in yellow or brownish cast, which can additionally darken by storage. We surprisingly found that the coloured impurities belong to at least three physico-chemical groups, which cannot be removed by only one technical operation. Critical impurities can be categorized into the following groups.

One group represents high molecular weight coloured impurities, which can be removed by methods of separation suitable for large molecules, such as adsorption to resins or gel filtration. They mainly comprise macromolecular residuals of cell structure.

Another group represents low molecular impurities, probably less than 1000 Da, of lower polarity that also originate from unspecific cell components comprising molecules with double bonded lipid parts.

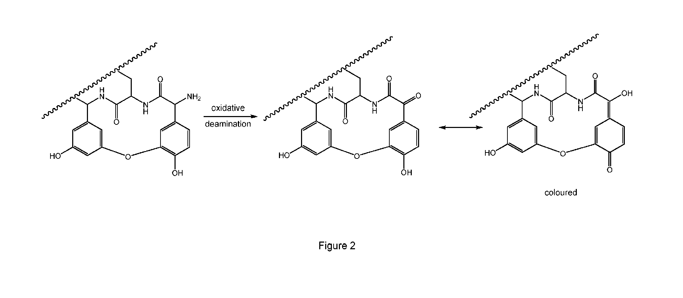

The third group represents closely related degradation products, characterised in the publication J. Antibiotics 49 (1996), p. 644-650, supposedly produced by oxidative aminolysis. Replacement of the amino group by a keto group causes a double effect: the affected part of the molecule becomes a conjugative quinone-like structure, moving the absorption maximum to the visual spectrum and the molecule loses basic character (Figure 2).

Thus, the invention provides a process for preparing purified teicoplanin, wherein according to one technical solution the process comprises subjecting teicoplanin to combined purification steps involving both, a cation exchange technique and an anion exchange technique. According to a further technical solution, the cation and/or the anion exchange resin, to which teicoplanin is bound, is eluted with an eluant having a pH from 5 to 9, preferably from 5 to below 9, which is preferably buffered by e.g. an organic buffer solution or at least one ionic compound.

A process for an efficient removal of impurities by means of adsorption of particular dissolved components from the filtrate of fermentation broth of *Actinoplanes teichomyceticus* culture is provided, which optionally comprises further purification steps such as adsorption of teicoplanin on commercial hydrophobic or slightly polar adsorbent resins from predominantly aqueous media and further removal of the product from the resin by means of a less polar eluant, mainly an eluant with reduced water content.

The filtrate of fermentation broth of *Actinoplanes teichomyceticus* culture herein means a filtered culture broth without mycelium prepared by procedures known to a skilled biotechnologist.

In a preferred embodiment of the invention, the steps of substantially removing coloured material from crude teicoplanin, the binding to a cation exchange resin and the adsorption to an anion exchange resin are combined. In this preferred embodiment, teicoplanin is prepared by:
a) providing crude teicoplanin in solution;
b) binding teicoplanin from the solution to a cation exchange resin and eluting the product with a buffer or a weak acid salt solution;
c) treating the obtained solution by routine methods of concentration and optional desalination, and binding teicoplanin from the solution to an anion exchange resin and eluting the product with a buffer solution or a weak acid salt solution;
d) submitting the obtained solution to a treatment with routine methods of isolation to remove impurities, salts and pyrogens;
e) isolating the final product with reduced amounts of coloured material.

In another option of this part of the invention teicoplanin is prepared, according to a preferred embodiment, by:
a') providing crude teicoplanin in solution;
b') binding teicoplanin from the solution to an anion exchange resin and eluting the product with a buffer or a weak acid salt solution;
c') treating the obtained solution by routine methods of concentration and optional desalination, and binding teicoplanin from the solution to a cation exchange resin and eluting the product with a buffer solution or a weak acid salt solution;
d') submitting the obtained solution to a treatment with routine methods of isolation to remove impurities, salts and pyrogens;
e') isolating the final product with reduced amounts of coloured material.

Providing crude teicoplanin solution of items a) and a') may include at least one of the following operations:
a1) incubating a fermentation culture of a strain of *Actinoplanes teichomyceticus* to give optimal concentration of teicoplanin compounds followed by removal of all undissolved material by filtration, ultrafiltration or centrifugation;
a2) adsorbing teicoplanin to a hydrophobic adsorption resin followed by washing the resin and elution of the product from the resin with a mixture of water and a water-miscible solvent, preferably methanol;
a3) treating the solution of teicoplanin with charcoal;
a4) concentrating and desalinating the solution by ultrafiltration on membranes with a cut-off of at least 1000 Da;
a5) isolating a solid crude product from the solution of any of the items a1) to a4) by evaporation and/or precipitation with an antisolvent, followed by redissolving the crude product in a medium appropriate in order to continue with the steps of items b) or b');
a6) taking coloured teicoplanin of any source, including a commercial source, and dissolving it in an appropriate solvent in order to continue with the steps of items b) or b').

Binding teicoplanin from the solution according to items b) and c') may include using the solution from the previous step a) or b'), adjusting the solution to conditions suitable for adsorption by optional partial evaporation, addition of a correcting solvent or adjusting pH and treating the obtained solution by mixing it with a cation exchange resin by stirring, removing medium from the resin by filtration followed by washing and desorption with an eluting medium or by adding the solution to a column of a cation exchange resin followed by washing and eluting the product by an eluting medium, wherein the cation exchange resin is preferably selected from macroreticular weak acid cation exchange resins, selected from, but not limited to, Amberlite^{®} IRC, Amberlite^{®} FPC, Diaion^{®} WK, Purolite^{®} C trademark groups, more preferably from Amberlite IRC50™, Amberlite FPC 3500™, Diaion WK 100™ (most preferably from Amberlite IRC50™) in a protonic form, and wherein the eluant preferably comprises a 10-40 % solution of water-miscible solvent in water, more preferably methanolic solution, most preferably 20 % methanol and an added buffer of pH in the range from 5 to 9, more preferably from 5 to below 9, or a solution of a salt of a weak acid, preferably alkali salt of lower aliphatic acid, most preferably of sodium acetate in a concentration range of 0.01-0.5 mol/L, for example 0.1M sodium acetate.

Binding teicoplanin from the solution according to items c) and b') may include using the solution from the previous step b) or a'), adjusting the solution to fit the optimal conditions suitable for adsorption by optional partial evaporation, addition of a correcting solvent or correcting pH and treating the obtained solution by mixing it with an anion exchange resin by stirring, removing medium from the resin by filtration followed by washing and desorption with an eluting medium or by pouring adding the solution to a column of an anion exchange resin followed by washing and eluting the product by an eluting medium wherein an anion exchange resin is preferably selected from, but not limited to, trademarks Amberlite^{®} FPA, Dowex^{®} Marathon, Dowex^{®} Upcore, Diaion^{®} WA, Purolite^{®} A, preferably Amberlite FPA51™, Amberlite FPA55™, Dowex Marathon WBA™, Purolite A830™, most preferably from Purolite A830™, and wherein the eluant preferably comprises 10-50 % solution of a water-miscible solvent in water, more preferably methanolic solution, most preferably 40 % methanol and an added a buffer of pH in the range from 5 to 9, preferably from 5 to below 9, or a solution of a salt of a weak acid, preferably alkali salt of lower aliphatic acid, most preferably of sodium acetate in a concentration range of 0.01-0.5 mol/L, most preferably 0.1M sodium acetate.

The eluants of steps b/b' and c/c' can be identical or different, preferably they are identical and contain at least one ionic compound, preferably selected from salts of weak acids. During elution using said eluant, teicoplanin, which is bound to the cation exchange resin, is replaced by a cationic part of the eluant, selected from alkali metal cations, earth alkali metal cations, ammonium, mono-, di-, tri- or tetra-substituted ammonium, in which substituents are identical or different and selected from C₁-C₁₆-alkyl, aryl substituted C₁-C₄-alkyl, preferably the cation part of the compound is selected from alkali metal cation, most preferably Na⁺, and teicoplanin, bound to the anion exchange resin, is replaced by the anionic part of the eluant, selected from anions of weak acids, particularly selected from phosphate, hydrogen phosphate, dihydrogen phosphate, carbonate, hydrogen carbonate, borate, organic C₁-C₁₆-containing linear or branched alkyl carboxylates, dicarboxylates, tricarboxylates or tetracarboxylates, wherein alkyl is optionally substituted with 1-3 hydroxyl groups, 1-2 amino groups, a mercapto or methylmercapto group or a phenyl group, most preferably the anionic part of the eluant is acetate and the solution of one or more ionic substances, which contains said cations and anions, exhibits a pH in the range from 5 to 9, preferably from 5 to below 9 ("below 9" means e.g. a pH of 8.8), more preferably in the range from 5.5 to 8.5, even more preferably from 6 to 8.5. Such eluant keeps teicoplanin in the pH range, in which it is most stable.

The combination of both, a cation and an anion exchange technique, has unexpectedly been found to lead to increased purity of the obtained teicoplanin, especially with respect to coloured impurities.

It has further surprisingly been found by the inventors that the use of a cation or anion exchange technique in combination with the specific elution conditions from the cation and or anion exchange resin according to the invention is particularly effective in providing teicoplanin that is characterized by a reduced amount of coloured impurities.

Application of further methods of isolation in order to remove impurities, salts and pyrogens according to item d) or d') may include at least one of the following methods
d1) treating the solution of teicoplanin with charcoal;
d2) removing last traces of biomacromolecules including pyrogens by ultrafiltration on membranes with a cut-off of at least 5000 Da;
d3) concentrating and desalinating the solution by ultrafiltration on membranes with a cut-off of at least 1000 Da;
d4) removing salts and last traces of biomacromolecules by an adsorption/desorption process on hydrophobic resins
d5) extraction of impurities by a water immiscible organic solvent
d6) concentration of the solution, optionally with partial exchange of solvents.

Isolation of the final product according to items e) or e') may include one of the following methods:
e1) spray drying the solution;
e2) lyophilisation of the solution;
e3) evaporation of the solvent, preferably at reduced pressure
e4) precipitation with an antisolvent and/or with pH adjustment,
wherein the most preferred method includes the option e4), precipitation with an antisolvent, selected from water-miscible solvents such as C₃-C₄-alcohols, C₃-C₄-ketones or C₂-C₄ nitriles, preferably acetone in 5-15 times larger volume, preferably in 10 times larger volume than the volume of teicoplanin solution. The precipitated product is isolated by filtration or centrifugation and dried *in vacuo* to yield white powder.

After any step, selected from a) to d), a') to d'), a1) to a4) and d1) to d6), pH of the solution is optionally adjusted to the value at which teicoplanin is most stable, preferably from 6.5 to 7.5, most preferably from 6.7 to 7.1, preferably by NaOH or HCl.

Application of the cation exchange technique or the anion exchange technique, combined with methods selected from a1) to a4), d1) to d6) and e4) reduces the level of impurities in a final powder to the pharmaceutically acceptable level and colours to a level, defined by transmittance of a water solution of 40 g/l at wavelength 405 nm of at least 40 %, while combining both ion exchange techniques reduces colours to a level defined by a transmittance of more than 80 % at the same conditions of measurement. According to the present invention, by the combination of both, cation and anion exchange chromatography the purity of the teicoplanin product is thus advantageously and unexpectedly enhanced. The combined use of anion and cation exchange techniques, even without an additional refined purification treatment such as charcoal treatment (see, for instance, Example 5) surprisingly allows for obtaining teicoplanin having a remarkably higher transmittance than, for example, teicoplanin obtained by employing only either cation or anion exchange technique in combination with such an additional purification treatment (e.g. charcoal treatment; see, for instance, Examples 3 and 4).

Using the process of the invention, teicoplanin can thus be provided having a transmittance of more than 80 % at a wavelength of 405 nm upon dissolution in water at a concentration of 40 g/l.

In order to reach optimal benefits of the process of the invention some pre-preparation operations can be applied depending on the purity of the source of teicoplanin. In the operation according to item a) suitable and commercially available hydrophobic or slightly polar adsorbent resins are selected from polystyrene or polyacrylate crosslinked non-ionic macroreticular resins having a pore radius of 20 to 500 A, selected from the group of trademarks, but not limited to Hypersol-Macronet®, Amberlite® XAD, Diaion® HP and Sepabeads SP, or Dowex® Optipore, preferably selected from the resins having a pore radius of 100 to 500 A selected from trademarks Amberlite® XAD16, Amberlite® XAD 1600, Amberlite® XAD 1180, Diaion® HP20, Diaion® Sepabeads SP207, Dianion® HP2MG, most preferably from Amberlite® XAD16, Amberlite® XAD 1600, Amberlite® XAD 1180. Resins of such type selectively adsorb molecules with lipophilic residues, including molecules of slightly larger molecular weight, such as teicoplanin, from aqueous solutions and leave low molecular polar components including ionic solutes and a variety of macromolecules. The less polar molecules are adsorbed from an aqueous solution, which contains no more than 30 % (v/v) of water-miscible solvents, preferably no more than 20 % of C₁-C₄-alcohols, acetone or acetonitrile, preferably of methanol.

The adsorbent binds and thereby removes, from the solution, at least 80 % of total teicoplanin, preferably at least 90 %, most preferably at least 95 % of total teicoplanin. The adsorption process is performed by shaking the resin suspended in said teicoplanin solution for at least 2 hours, preferably at least 12 hours, most preferably at least 24 hours at temperature from 0-40 °C, preferably at room temperature. Finally the teicoplanin containing resin is filtered off and optionally washed with water by resuspending or rinsing. Alternatively the teicoplanin containing filtered broth is eluted through a column of said adsorbent up to the saturation of the adsorbent with fermentation products.

The adsorbed species are released from the resins by eluting with aqueous solutions rich in organic solvent, preferably containing at least 40 % (v/v) of a water-miscible organic solvent, selected from C₁-C₄-alcohols, acetone or acetonitrile, preferably of methanol. The separation of teicoplanin from impurities can be improved by continuous elution from a column packed with a teicoplanin loaded resin, when the process is led similarly to a chromatographic process, in which teicoplanin is preferably eluted in different fractions. The separation of teicoplanin may be improved by longer columns but there should be a compromise between efficiency of separation and eluting solvent volume, which can enhance costs and technical applicability. Nevertheless, a complete removal of related substances and of a variety of coloured impurities cannot be achieved only by this step.

In an embodiment of the invention, the eluted solution of teicoplanin is optionally pH-adjusted to 6-7 and then concentrated to 2 - 20 % (w/v), preferably to 5 - 15 % (w/v), most preferably to 10 % (w/v) of teicoplanin content, followed by addition of an antisolvent, selected from water-miscible solvents such as C₃-C₄-alcohols, C₃-C₄-ketones or C₂-C₄-nitriles, preferably from acetone in 5-15 times larger volume, preferably in 10 times larger volume than the volume of a teicoplanin solution. The precipitate is separated by filtration or centrifugation to yield crude yellow or brown product.

In a further preferred embodiment, the eluted solution of teicoplanin is pH-adjusted to 6-7, optionally diluted with water or a water-miscible solvent and further treated by active charcoal. The amount of charcoal is added in 5 - 300 % (w/w), preferably in 50 - 100 % (w/w) of an expected amount of teicoplanin in the solution, and the mixture is treated for 5 minutes to 12 hours, preferably to 0.5 - 2 hours at 0-60 °C, preferably at room temperature. The active charcoal is then filtered off and the solution of teicoplanin is concentrated to 2 - 20 % (w/v), preferably to 5 - 15 % (w/v), most preferably to 10 % (w/v) of teicoplanin content, followed by addition of an antisolvent, selected from water-miscible solvents such as C₃-C₄-alcohols, C₃-C₄-ketones or C₂-C₄-nitriles, preferably from acetone in 5-15 times larger volume, preferably in 10 times larger volume than the volume of the teicoplanin solution. The precipitate is separated by filtration or centrifugation to yield crude yellow product.

Such adsorption/desorption process on hydrophobic resins removes most of fermentation residues, a variety of macromolecular impurities and polar low molecular residuals but not more related impurities and nonpolar residuals, especially some coloured material. Even more, surprisingly the charcoal treatment is not as efficient as expected in removing colours, thus creating a need for larger volumes of the adsorbent.

In an embodiment of this invention, crude teicoplanin that is prepared by purification on hydrophobic resins and/or on active charcoal or any other method known for isolation of fermentation products, but that still contains considerable amounts of coloured material, is purified by binding to a cation exchange resin with further elution with an eluting solution. In another embodiment, teicoplanin is bound to the resin by adding the resin to the stirred or shaken solution followed by filtering the resin, washing it and releasing the product by treating with an eluting solution. Preferably the cation exchange resin treatment is performed in a column like as it is applied in a chromatography. The cation exchange resin is preferably selected from an acid cation exchange resin, preferably weak acid cation exchange resins, more preferably from macroreticular weak acid cation exchange resins selected from, but not limited to, Amberlite^{®} IRC, Amberlite^{®} FPC, Diaion^{®} WK, Purolite^{®} C trademark groups, more preferably from Amberlite IRC50™, Amberlite FPC 3500™, Diaion WK 100™, most preferably from Amberlite IRC50™ in a protonic form.

In another embodiment of this invention, crude teicoplanin, that is prepared by purification on hydrophobic resins and/or on active charcoal or any other method known for isolation of fermentation products, but that still contains considerable amounts of coloured material, is purified by binding to an anion exchange resin with further elution with an eluting solution. In an embodiment of the invention, teicoplanin is bound to the resin by adding the resin to the stirred or shaken solution followed by filtering the resin, washing it and releasing the product by treating with an eluting solution. Preferably the anion exchange resin treatment is performed on a column as it is applied in chromatography. The anion exchange resin is preferebly selected from basic anion exchange resins, preferably weak basic anion exchange resins, more preferably from macroreticular weak basic anion exchange resins, selected from, but not limited to, trademarks Amberlite^{®} FPA, Dowex^{®} Marathon, Dowex^{®} Upcore, Diaion^{®} WA, Purolite^{®} A, preferably Amberlite FPA51™, Amberlite FPA55™, Dowex Marathon WBA™, Purolite A830™, most preferably from Purolite A830™.

The eluant preferably comprises 10-50 %, in the case of cation exchange technique 10-40 %, of a water-miscible solvent in water, preferably methanolic solution, most preferably 20 % methanol.

In a preferred embodiment, the eluant comprises a pH buffer, which provides for a pH value in the range from 5 to 9, preferably a pH from 5 and to below 9, more preferably in the range from 5.5 to 8.5, even more preferably from 6 to 8.5. In particular, the eluant may further comprise an organic buffer such as, for example, Tris/HCl, Bicine, Tricine or HEPES, or the like, thus ensuring a suitable pH value of the eluant.

In a preferred embodiment, the eluant comprises an ionic compound, wherein the ionic compound is a cation selected from alkali metal cations; earth alkali metal cations; ammonium; mono-, di-, tri- or tetra-substituted ammonium, in which substituents are identical or different and selected from C₁-C₁₆-alkyl, aryl, substituted C₁-C₄-alkyl. In a particular embodiment, the cation is an alkali metal cation.

The ionic compound can further be an anion of a weak acid selected from the group consisting of phosphate; hydrogen phosphate; dihydrogen phosphate; carbonate; hydrogen carbonate; borate; organic C₁-C₁₆-containing linear or branched alkyl carboxylates, dicarboxylates, tricarboxylates or tetracarboxylates, wherein alkyl is optionally substituted with 1-3 hydroxy groups, 1-2 amino groups, a mercapto or methylmercapto group or a phenyl group. In a preferred embodiment, the anion is acetate.

Thus, in a particular embodiment of the invention, a solution of a salt of a weak acid is used in order to stabilize the pH value as mentioned above, preferably an alkali salt of a lower aliphatic acid, most preferably of sodium acetate in a concentration range of 0.01-0.5 mol/L, for example of 0.1M sodium acetate.

The added buffer or solution is relevant for easy elution and continuous protection of sensitive molecules of teicoplanin from potential extreme pH values caused by elution of protons from unoccupied active sites of ion exchange in a cation exchange technique. On the other hand, in the case of an anion exchange technique, it is thus provided that the pH-sensitive teicoplanin is protected from overalkalisation of the eluted solution, which would cause considerable epimerisation of the molecule. Therefore, the added buffer or solution significantly contributes to obtaining a unique combination of improved yields of teicoplanin and substantially reduced impurities, in particular, coloured impurities.

The colourless or slightly yellowish fractions, which contain teicoplanin, are combined, optionally treated to remove last traces of macromolecules and pyrogens and finally desalinated by methods known to a skilled person, such as adsorption/desorption process on adsorption resins (for example as described above with respect to pre-preparation operation a2)), filtration through membranes, or ultrafiltration. The obtained solution can be concentrated to 2 - 20 % (w/v), preferably to 5 - 15 % (w/v), most preferably to 10 % (w/v) of teicoplanin content, optionally adjusting the solvent ratio to have 0-20 % (v/v) of an aqueous, water-miscible organic solvent, preferably methanol, followed by addition of an antisolvent, selected from water-miscible solvents such as C₃-C₄-alcohols, C₃-C₄-ketones or C₂-C₄ nitriles, preferably from acetone in 5-15 times larger volume, preferably in 10 times larger volume than the volume of teicoplanin solution. A formed precipitate can be separated, e.g. by filtration or centrifugation, to yield almost white or slightly yellowish powder.

In a preferred embodiment the solution of crude teicoplanin is previously prepared by dissolving of any solid crude teicoplanin, including insufficiently pure material from a commercial source, in a mixture of a water-miscible solvent in water, preferably in a methanolic solution, most preferably in 20 % (v/v) methanol, followed by said treatment by adsorption on a cation exchange resin. In another embodiment a solution of teicoplanin can be directly transferred from a previous process step, obtained after filtering off mycelium and other insoluble material from a fermentation broth and/or after treatment by adsorption on hydrophobic resin as described above and/or after treatment by adsorption on active charcoal. The solution from said steps can be optionally concentrated and adjusted to obtain 10-40 % (v/v) solution of a water-miscible solvent in water, preferably to obtain 10-40 % (v/v) methanolic solution, most preferably 20 % (v/v) methanol, followed by said treatment by adsorption on a cation exchange resin. Such treatment substantially removes coloured impurities with a weak or no affinity to a cation exchange resin and/or has at least essentially different affinity compared to teicoplanin. Such treatment preferably removes non-basic impurities, preferably oxidised coloured impurities, which are closely related to teicoplanin, but they do not contain the basic amino group, important for a stronger binding to the cation exchange resin.

Coloured impurities having weak or no affinity to an anion exchange resin can be separated from teicoplanin in an analogous manner to the one above by (additional) use of an anion exchange technique.

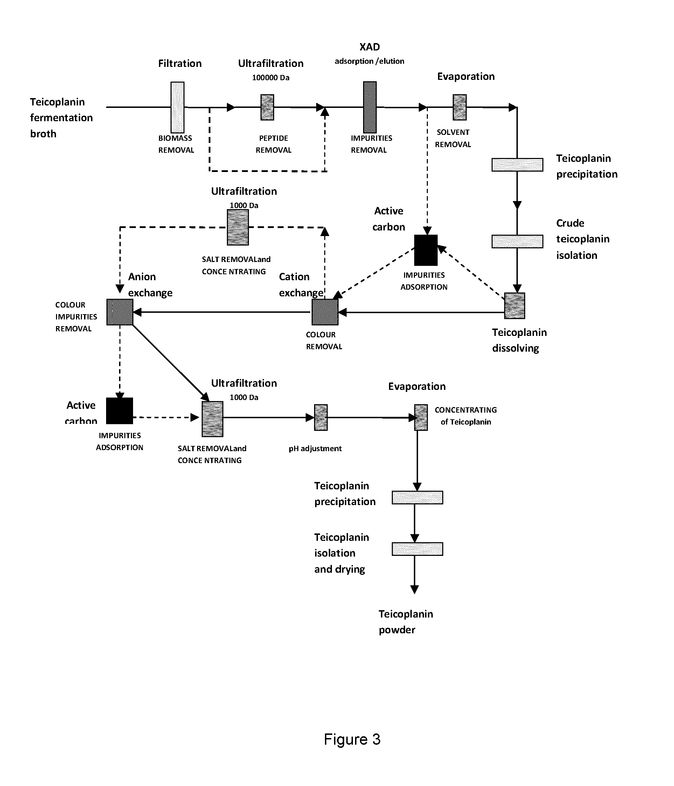

A particularly preferred embodiment of an overall process of the invention is schematically shown in Figure 3. The hatched lines therein represent optional operations.

The preparation of a pharmaceutical composition containing teicoplanin of high purity and pharmaceutically acceptable excipients is a further aspect of the present invention. A pharmaceutical composition according to the invention is suitable for parenteral administration and the therapeutic dose of teicoplanin according to the invention in any given case will depend on the nature and severity of the disease to be treated. The dose and dose frequency may also vary according to the age, body weight, and response of the individual patient. In general, a suitable dose of the active ingredient is within the range of 50 mg to 800 mg daily, preferably 100 mg to 400 mg, preferably between 100 to 800 mg of total daily dosage. Dosage forms include dispersions, solutions, suspensions, emulsions, powders, implants, wherein the most preferred dosage forms of teicoplanin are dry powder injections and infusions. In a most preferred embodiment the novel teicoplanin described herein is dissolved in sterile and apyrogen water together with stabilisers, buffers, and additives, and the mixture is lyophilised to give amorphous powder, which is reconstituted in prepared liquid just before use. The reconstituted injection may be administered either intravenously or intramuscularly. The intravenous injection may be administered, for example, as a bolus or as a 30 minute infusion. Dosage is usually once daily but, in cases of severe infection, a second injection should be administered on the first day in order to reach the required serum concentrations more rapidly.

### Examples

The following examples further illustrate the invention. They are provided for illustrative purposes only and are not intended to limit the invention, in any manner.

The amounts of teicoplanin components in various intermediate solutions or isolated solid intermediates and final products of representative experimental examples are measured by HPLC method on Agilent 1100™ instrument. The mobile phase A contains a 5 mM phosphate buffer of pH 4.9 and the mobile phase B contains acetonitrile. Chromatographic peaks were detected at 280 nm, temperature of column was 45 °C and 5 µl of sample was injected. The separation was carried out on the column Ascentis Express C18™, 10 cm x 4.6 mm, 2.7 µm equipped with precolumn C18, 2 cm x 4.0 mm, 3 µm. Flow rate of 2.2 ml/min and the gradient during chromatography was as follows:

**Table1**

| Time (min) | Mobile phase B |
|---|---|
| 0.0 | 15% |
| 0.8 | 20 % |
| 0.9 | 24 % |
| 3.3 | 25 % |
| 4.8 | 29 % |
| 8.4 | 41 % |
| 8.7 | 60 % |
| 9.2 | 60 % |
| 9.5 | 15% |
| 11.0 | 15% |

Values are expressed as area percentage of the sum of all teicoplanin components or particular components themselves and have mainly relative or comparative meaning.

Transmittance is measured on Perkin Elmer Lamda 25 UV-VIS spectrometer at 405 nm and at concentration of teicoplanin samples of 40 g/l in water.

### EXAMPLE 1

### Isolation of crude fermentation broth

Fresh whole broth, collected immediately after the end of fermentation of *Actinoplanes teichomyceticus* was used for experiments. A broth supernatant (clarified broth) was produced by centrifuging the broth in a laboratory centrifuge (Megafuge 1.0R™, Heraeus) at 4000-6000 rpm for 10-30 minutes. The supernatant was additionally filtered through a sintered glass filter funnel (porosity 4 and 5, Schott Duran GmbH) and later analyzed by means of HPLC method. Clear filtrate (permeate) was produced by means of a cross-flow filtration. Ceramic filtration module (Membralox 1P19-60™) with 0.1 µm pore size was used. Permeate was collected and analyzed by means of HPLC method.

### EXAMPLE 2

### Preparation of crude teicoplanin

70 L of culture broth of *Actinoplanes teichomyceticus* were diluted with 70 L of demineralized water and filtered using vacuum filter to remove mycelium obtaining 140 L of filtrate. The broth was analyzed by an RP-C18 (4.6x250 mm) HPLC column. With respect to this, the content and total amount of teicoplanin in the broth were from 2 - 3 g/L. 35 L of methanol were added to 140 L of filtrate, and the resulting mixture was applied onto a column packed with 11 L of Amberlite XAD 16 at a flow rate of 0.3 BV (bed volume)/hr. Subsequently, 8 BV (90 L) of demineralized water were loaded at a flow rate of 2 BV/hr onto the column to wash the resin packed in the column. Afterwards 9 BV (100 L) of 30% (v/v) methanol were loaded at a flow rate of 0.5 BV/hr onto the column to wash the resin packed on the column. Further 4.5 BV (50 L) of 85% (v/v) methanol were loaded onto the column at a max flow rate of 0.5 BV/hr to elute teicoplanin. With respect to this, the content of total amount of teicoplanin in the eluting solution was 2,2 g/L.

Further 70 g of Norit CA1 as an activated carbon were added to 35 L of the above teicoplanin solution and the resulting mixture was stirred at room temperature for 1 hour, and then filtered to remove the activated carbon from the resulting mixture. The pH of the resulting filtrate, with the content of teicoplanin 1.59 g/L, was adjusted to 7.0 using NaOH. The filtrate was concentrated using a vacuum evaporation until 0.7 L of concentrate was reached. As well, 7 L of acetone were slowly added to the concentrate while the concentrate was agitated to precipitate teicoplanin for 3 hours. A portion (6.7 L) of resulting suspension was filtered by filter nuch B4 to recover a precipitate. The precipitate was dried in a vacuum drier at 40 °C for 21 hours to recover 46.3 g of crude teicoplanin powder (83% purity). Teicoplanin powder was dissolved in distilled water to provide a concentration of teicoplanin of 40 mg/ml. Transmittance at 405 nm of a teicoplanin solution was evaluated for removal of coloured components. In consequence, the teicoplanin powder of the present invention had a transmittance of below 17 %.

### EXAMPLE 3

### Decolourisation using cation exchange resin

7,3 g of Norit CA1 as an activated carbon were added to 3,3 L of teicoplanin eluting solution with 2,2 g/L of teicoplanin, prepared according to the first paragraph of the description in Example 2. The resulting mixture was stirred at room temperature for 1 hour, and then filtered to remove the activated carbon from the resulting mixture. The pH of the resulting filtrate, with the content of teicoplanin 1.7 g/L, was adjusted to pH 6,7 using NaOH. Afterwards filtrate was dissolved with 10,6 L of demineralized water and so prepared solution was applied onto a column packed with 0,54 L of Amberlite IRC50 (cation exchange resin) at a flow rate of 0,65 BV /hr. Subsequently, 3 BV (1,5 L) of 20% (v/v) methanol were loaded at a flow rate of 2 BV/hr onto the column to wash the resin packed on the column. Further 7,6 BV (4,1 L) of 20% (v/v) methanol solution containing 1 mol of Na-acetate were loaded onto the column at a max flow rate of 2 BV/hr to elute teicoplanin. The pH of eluted teicoplanin was adjusted to 6,9 using NaOH and the whole amount (4,1 L) of eluted teicoplanin solution was further desalinated by adsorbing the product to 0.1 L of Amberlite XAD16 and eluting it with 85% (v/v) methanol. Afterwards 0,475 L of desalinated 85% (v/v) methanol solution of teicoplanin were obtained (more than 98 area % HPLC purity). With respect to this, the content and total amount of teicoplanin in the elute were 2.6 g/L and 1,05 g, respectively. Further 2 g of Norit CA1 as an activated carbon were added to 0.4 L of the above teicoplanin solution and the resulting mixture was agitated at room temperature for 1 hour, and then filtered to remove the activated carbon from the resulting mixture. The pH of the resulting filtrate, with the content of teicoplanin was adjusted to 7.3 using NaOH. The filtrate was concentrated using a vacuum evaporation until 10 ml of concentrate was reached. The pH of the resulting concentrate was adjusted to 6,9 using NaOH. Afterwards 120 mL of acetone were slowly added to the stirred mixture to precipitate teicoplanin. The suspension was stirred for further 3 hours and the resulting suspension was filtered through a filter nuch B4 to recover a precipitate. The precipitate was dried in a vacuum drier at 40 °C for 20 hours to recover 0.8 g of teicoplanin powder (more than 98 area % HPLC purity).

Teicoplanin powder was dissolved in distilled water to provide a concentration of teicoplanin of 40 mg/ml. Transmittance at 405 nm of a teicoplanin solution was evaluated for removal of coloured components. In consequence, the teicoplanin powder of the present invention had a transmittance of 44 %.

### EXAMPLE 4

### Decolourisation using anion exchange resin

1,3 g of Norit CA1 as an activated carbon were added to 0,6 L of teicoplanin eluting solution with 2,17 g/L of teicoplanin, prepared according to the first paragraph of the description in Example 2. The resulting mixture was stirred at room temperature for 1 hour, and then filtered to remove the activated carbon from the resulting mixture. The pH of the resulting filtrate, with the content of teicoplanin 1.7 g/L, was adjusted to pH 6,7 using NaOH. Afterwards filtrate was dissolved with 0,7 L of demineralized water and so prepared solution was applied onto a column packed with 0,1 L of Purolite A830 (anion exchange resin) at a flow rate of 0,25 BV/hr. Subsequently, 3 BV (0,3 L) of 40% (v/v) methanol were loaded at a flow rate of 2.5 BV/hr onto the column to wash the resin packed on the column. Further 3 BV (0,3 L) of 40% (v/v) methanol solution containing 1 mol of Na-acetate were loaded onto the column at a max flow rate of 2 BV/hr to elute teicoplanin. The pH of eluted teicoplanin was adjusted to 6,9 using NaOH and the whole amount (0,3 L) of eluted teicoplanin solution was further on diluted with 0,3 L of demineralized water and desalinated by adsorbing the product to 0.02 L of Amberlite XAD16 and eluting it with 85% (v/v) methanol. Afterwards 0,116 L of desalinated 85% (v/v) methanol solution of teicoplanin was obtained (with more than 90 area % HPLC purity). With respect to this, the content and total amount of teicoplanin in the eluate were 4,31 g/L and 0,5 g, respectively. Further 1 g of Norit CA1 as an activated carbon was added to 0.116 L of the above teicoplanin solution and the resulting mixture was agitated at room temperature for 1 hour, and then filtered to remove the activated carbon from the resulting mixture. The pH of the resulting filtrate, with the content of teicoplanin was adjusted to pH 7.0 using NaOH. The filtrate was concentrated using a vacuum evaporation until 4 ml of concentrate was reached. The pH of the resulting concentrate was adjusted to 6,9 using NaOH. Afterwards 50 mL of acetone were slowly added to the stirred mixture to precipitate teicoplanin. The suspension was stirred for further 3 hours and the resulting suspension was filtered through a filter nuch B4 to recover a precipitate. The precipitate was dried in a vacuum drier at 40 °C for 20 hours to recover 0.38 g of teicoplanin powder (more than 90 area % HPLC purity).

Teicoplanin powder was dissolved in distilled water to provide a concentration of teicoplanin of 40 mg/ml. Transmittance at 405 nm of a teicoplanin solution was evaluated for removal of coloured components. In consequence, the teicoplanin powder of the present invention had a transmittance of 42 %.

### EXAMPLE 5

### Preparation of pure teicoplanin

45 g of crude teicoplanin powder according to Example 2 were dissolved in 30 L of 20% (v/v) methanol and the so prepared solution was applied onto a column packed with 3 L of Amberlite IRC50 (cation exchange resin) at a flow rate of 0,65 BV /hr. Subsequently, 4 BV (12 L) of 20% (v/v) methanol were loaded at a flow rate of 2 BV/hr onto the column to wash the resin packed on the column. Further 10 BV (30 L) of 20% (v/v) methanol solution containing 1 mol of sodium acetate was loaded onto the column at a max flow rate of 2 BV/hr to elute teicoplanin. The pH of eluted teicoplanin was adjusted to 6,7 using NaOH and the whole amount (30L) of eluted teicoplanin solution was further desalinated by adsorbing the product to 0.7 L of Amberlite XAD16 and eluting it with 85% (v/v) methanol. Afterwards 5.4 L of desalinated 85% (v/v) methanol solution of teicoplanin were obtained (more than 98 area % HPLC purity) and the pH of eluted teicoplanin was adjusted to 7,1 using NaOH. With respect to this, the content and total amount of teicoplanin in the eluate were 4.3 g/L and 23.22 g, respectively.

1 L of desalinated 85% (v/v) methanol solution of teicoplanin, prepared as described above was diluted with 1 L of demineralized water and so prepared solution was applied onto a column packed with 0,5 L of Purolite A830 (anion exchange resin) at a flow rate of 0,25 BV/hr. Subsequently, 4 BV (2 L) of 40% (v/v) methanol were loaded at the flow rate of 2.5 BV/hr onto the column to wash the resin packed on the column. Further, 4 BV (2 L) of 40% (v/v) methanol solution containing 1 mol of sodium acetate were loaded onto the column at a max flow rate of 2 BV/hr to elute teicoplanin. The pH of eluted teicoplanin was adjusted to 6,8 using NaOH and the whole amount (2 L) of eluted teicoplanin solution was further on diluted with 2L of demineralized water and desalinated by adsorbing the product to 0.1 L of Amberlite XAD16 and eluting it with 85% (v/v) methanol. Afterwards 0.45 L of desalinated 85% (v/v) methanol solution of teicoplanin was obtained (with more than 98 area % HPLC purity). With respect to this, the content and total amount of teicoplanin in the eluate was 4.58 g/L and 2.0 g, respectively. 0.15 L of desalinated teicoplanin solution was than concentrated using a vacuum evaporation until 10 ml of concentrate were reached. The pH of concentrate was adjusted to 7.0 and 120 mL of acetone were slowly added to the stirred mixture to precipitate teicoplanin. The suspension was stirred for further 3 hours and the resulting suspension was filtered by filter nuch B4 to recover a precipitate. The precipitate was dried in a vacuum drier at 40 °C for 20 hours to recover 0.52 g of teicoplanin powder (with more than 98 area % HPLC purity).

Teicoplanin powder was dissolved in distilled water to provide a concentration of teicoplanin of 40 mg/ml. Transmittance at 405 nm of a teicoplanin solution was evaluated for removal of coloured components. In consequence, the teicoplanin powder of the present invention had, as measured in the indicated reference solution, a transmittance of 81 %.

## Claims

1. A process for preparing purified teicoplanin, the process comprising subjecting teicoplanin to combined purification steps involving both, a cation exchange technique and an anion exchange technique.

2. A process according to claim 1 comprising the steps, in the following indicated order, of either
a) providing teicoplanin in solution;
b) binding teicoplanin from the solution to a cation exchange resin, followed by eluting with an eluant;
c) binding teicoplanin to an anion exchange resin, followed by eluting with an eluant, and
d) obtaining purified teicoplanin;
or
a') providing crude teicoplanin in solution;
b') binding teicoplanin from the solution to an anion exchange resin, followed by eluting with an eluant;
c') binding teicoplanin to a cation exchange resin, followed by eluting with an eluant, and
d') obtaining purified teicoplanin.

3. A process for preparing purified teicoplanin, the process comprising subjecting teicoplanin to a cation or to an anion exchange technique, wherein either a cation or an anion exchange resin having teicoplanin bound thereto is treated with an eluant having a pH value in the range from 5 to below 9.

4. The process according to claim 3, wherein the eluant has a pH value in the range from 5.5 to 8.5, more preferably from 6 to 8.5.

5. The process according to any one of the preceding claims, wherein teicoplanin is previously provided by a process, which includes at least one of the following steps, preferably a combination of the indicated steps, of
a1) incubating a fermentation culture of a strain of *Actinoplanes teichomyceticus* producing teicoplanin compounds, followed by removal of undissolved material by filtration, ultrafiltration or centrifugation;
a2) adsorbing teicoplanin to a hydrophobic adsorption resin, optionally followed by washing the resin, and elution from the resin by a mixture of water and water-miscible solvent, wherein preferably the water-miscible solvent is methanol;
a3) treating a teicoplanin sample with charcoal;
a4) concentrating and desalinating a solution of teicoplanin by ultrafiltration on a membrane with a cut-off of at least 1000 Da.

6. The process according to any one of the preceding claims, wherein a cation exchange resin is used, which is a macroreticular weak acid cation exchange resin selected from the group consisting of Amberlite^{®} IRC, Amberlite^{®} FPC, Diaion^{®} WK and Purolite^{®} C trademark groups and/or an anion exchange resin is used, which is selected from the group consisting of Amberlite^{®} FPA, Dowex^{®} Marathon, Dowex^{®} Upcore, Diaion^{®} WA and Purolite^{®} A trademark groups.

7. The process according to any one of the preceding claims, wherein the teicoplanin-containing sample is treated by at least one further method of purification before proceeding with the respectively other ion exchange technique, wherein preferably the at least one further method of purification comprises at least one selected from the group consisting of a treatment of the solution with charcoal; removing biomacromolecules by ultrafiltration using a membrane with a cut-off of at least 5000 Da; concentrating and desalinating the solution by ultrafiltration on a membrane with a cut-off of at least 1000 Da; removing salts and biomacromolecules by an absorption/desorption process on a hydrophobic adsorption resins; extraction of impurities by a water-immiscible organic solvent; concentrating the solution, optionally with partial exchange of solvents.

8. The process according to any one of the preceding claims, wherein purified teicoplanin is isolated by any method selected from spray drying; liophilisation; evaporation of the solvent, preferably at reduced pressure; or precipitation using an antisolvent and/or with pH adjustment.

9. The process according to any one of the preceding claims, wherein,
in the cation exchange technique, an eluant comprises 10-40 % of a water-miscible solvent in water, and
in the anion exchange technique an eluant comprises 10-50 % of a water-miscible solvent in water.

10. The process according to any one of the preceding claims, wherein, in the cation and/or the anion exchange technique, an eluant comprises an organic buffer or a solution of a salt of a weak acid.

11. The process according to any one of the preceding claims, wherein, in the cation and/or the anion exchange technique, an eluant comprises at least one ionic compound, preferably the ionic compound is a cation selected from alkali metal cations; earth alkali metal cations; ammonium; mono-, di-, tri- or tetra-substituted ammonium, in which substituents are identical or different and selected from C₁-C₁₆-alkyl, aryl, substituted C₁-C₄-alkyl, and/or
the ionic compound is an anion of a weak acid selected from the group consisting of phosphate; hydrogen phosphate; dihydrogen phosphate; carbonate; hydrogen carbonate; borate; organic C₁-C₁₆-containing linear or branched alkyl caboxylates, dicarboxylates, tricarboxylates or tetracarboxylates, wherein alkyl is optionally substituted with 1-3 hydroxy groups, 1-2 amino groups, a mercapto or methylmercapto group or a phenyl group.

12. The process according to any one of claim 10 or 11, wherein, in the cation and/or the anion exchange technique, the eluant comprises a solution of an alkali salt of a lower aliphatic acid.

13. Teicoplanin **characterized by** having a transmittance of more than 80 %, at wavelength 405 nm upon dissolution in water at a concentration of 40 g/l.

14. A pharmaceutical composition comprising the teicoplanin of claim 13.

15. A process for manufacturing a pharmaceutical composition comprising providing teicoplanin as defined in claim 13 and mixing the provided teicoplanin with at least one pharmaceutically acceptable excipient or carrier.
